# EUROPEAN PATENT SPECIFICATION

(11) **EP 0 345 228 B1**
(45) Date of publication and mention of the grant of the patent: **03.08.1994**
(21) Application number: 89830212.0
(22) Date of filing: 16.05.1989
(51) Int. Cl.: A61C 19/00, A61L 2/18

(54) **Apparatus for the sterilization and mechanical cleansing of medical, surgical and dental instruments such as handpieces, mirrors, bistouries and the like**
Vorrichtung für die Sterilisation und die mechanische Reinigung medizinischer, chirurgischer und zahnärztlicher Instrumente, wie Handstücke, Speigel, Messer und ähnliches
Appareil destiné à la stérilisation et le nettoyage mécanique d'instruments médicaux, chirurgicaux et dentaires, comme des pièces à main, miroirs, bistouris et analogues

(30) Priority: 17.05.1988 IT 346088
(43) Date of publication of application: 06.12.1989
(73) Proprietor: CASTELLINI S.p.A., I-40013 Castelmaggiore (Bologna) (IT)
(72) Inventor: Lenzi, Plinio Maria, I-40129 Bologna (IT); Sacchetti, Enzo, I-40141 Bologna (IT); Pignattini, Giorgio, I-40127 Bologna (IT); Lanzarini, Giuliano, I-40138 Bologna (IT)
(74) Representative: Lanzoni, Luciano

(56) References cited:
- EP-A- 0 111 249
- EP-A- 0 233 847
- EP-A- 0 321 415
- DE-C- 3 611 327
- US-A- 4 601 300

## Description

The present invention relates to an apparatus for the sterilization and mechanical cleansing of medical, surgical and dental instruments such as handpieces, mirrors, bistouries and other similar items; the scope of the invention also includes disinfection, which differs from full sterilization only as regards the length of time that the instruments remain in contact with the liquid medium utilized, and accordingly, the general term "sterilization", adopted throughout the specification for convenience, is intended to embrace both sterilization and disinfection.

The greatest single problem encountered by medical and paramedical personnel operating in small health structures, i.e. equipped essentially for minor surgery, is that connected with the sterilization of surgical instruments in regular use.

In order to obtain a thorough sterilization of such instruments, use is made currently of specifically designed equipment, for example the autoclave, in which the soiled instruments are placed and left for a given duration; sterilization is achieved by producing a substantial temperature rise internally of the vessel.

Clearly, considerable quantities of energy are required for the operation of such equipment, and moreover, not all instruments can be subjected to the same degree of heat, due to the fact of their being fashioned either in a thermolabile material, or with mechanisms that are highly delicate and thus extremely sensitive in operation, affected even by minimal dimensional variations attributable to the different coefficients of expansion of their component parts. The latter is particularly true, for instance, in the case of certain dental surgery instruments, such as turbine drills or microdrive handpieces, which are complex in construction and notably compact. Such instruments cannot be treated in an autoclave, but are immersed for an extended duration in liquid sterilizer, for example a 2% solution of glutaraldehyde.

Sterilizing systems have been developed recently, intended specifically for instruments utilized in dental surgery and designed for use together with conventional apparatus pedestals, which connect with the internal passages of drill handpieces incorporating spray air and water circuits; when such instruments are switched off, a vacuum is generated briefly through the water circuit to the end of preventing the handpieces from dripping.

For example, the pubblication DE-C-3 611 327 shows a container for handpieces in which the disinfecting fluid is simply contained into the bottom recipient which is provided by a central overflow outlet converging into the drainage pipe of the spitton bowl in such a way that the fluid leaving the instruments is conveyed into the existing dental apparatus spitton bowl drainage pipe. In said solution the container has the only object to support the internal disinfecting duct instruments and to define an holder able to retain the disinfecting fluid leaving the instruments and conveying the same towards the spitton bowl pipe.

The pubblication US-A-4 601 300 refers to a classic disinfecting apparatus for medical instruments in a method including the steps of placing the instruments in the inner vessel, washing the articles, draining the inner vessel and so applying the disinfectant to the articles. This solution expressly provides the utilization of an inner vessel and an outer vessel so shaped in order that an outer chamber is defined therebetween and the inner vessel is completely drained and submerged by the fluid contained into the outer vessel in ordel to realize the disinfection both of the articles and of the inner vessel.

With this arrangement, the internal fluid passages of an instrument can be exploited directly, and used to flood the circuits alternately with liquid sterilizer and with rinsing water; accordingly, a faultless sterilization is obtained internally of the passages. Alternatively to this solution, according to the EP-A-O 321 415, which is an earlier European application which is only to be considered under Art. 54(3) EPC for the purpose of novelty, a container is provided at its top by a plurality of sockets for the accomodation of the instruments to be sterilized whose bottom is shaped in order to fit exactly into the well normally occupied by the rinse drinking glass used by patients, the bottom of the container affords at least one outlet from which discharge of the soiled liquids occurs directly, preferably drawing out by the vacuum system of the surgery equipment with which the instruments are associated.

Nonetheless, even equipment operating by this method is able to solve the aforementioned sterilization problem only in part; more exactly, when using instruments that are designed to protect an atomized spray internally of the oral cavity, one has the risk that pollutant particles of blood, amalgam, debris etc., may be carried by the spray back onto the eternal surface of the end part of the instrument, or penetrate and accumulate in its recessed parts.

Thus it happens that when an instrument is returned to its holder following use, the particles dry out and form encrustations that cling hard and cannot be removed thereafter solely by a sterilization effected with the prior art equipment summarized above, whether from the external or the internal surfaces of the instrument.

As far as regards external encrustations, these are either removed by hand, or bombarded and broken up utilizing ultrasonic equipment. Such equipment is costly, however, and does no more than dislodge the encrustations, so that their final removal requires manual intervention just the same.

Accordingly, the object of the present invention is to provide an apparatus by means of which surgery instruments can be sterilized internally and externally, and cleansed, swiftly, efficiently and with proportionate economy.

The stated object is realized with an apparatus as characterized in the appended claims.

One advantage of the invention consists essentially in its operational and constructional simplicity, at least as far as the equipment is concerned, most especially in the case of instruments with water and air circuits such as a turbine drill, since the drill itself can be exploited both as a source of liquid sterilizer and as a means of creating the necessary turbulence in the liquid.

Another advantage of the invention is that of the compact dimensions of the equipment, which enables its successful utilization even in small surgery facilities without problems of available space.

The invention will now be described in detail, by way of example, with the aid of the accompanying drawings, in which:
fig 1 is a plan of the equipment disclosed, viewed with certain of the parts omitted;
fig 2 is the section through II-II in fig 1;
fig 3 shows a detail of the equipment of fig 1, seen in plan;
fig 4 is the section through IV-IV in fig 3;
The apparatus according to the invention uses a liquid sterilizing medium 2 in which soiled instruments 3 are immersed, at least in part, and envisages the use of means by which to generate a turbulent flow in the liquid sterilizing medium 2, in such a way that all the internal and/or external surfaces of the instruments 3 in contact with the liquid 2 and exposed to the turbulence are subjected to a mechanical cleansing action.

The liquid sterilizing medium 2, or more simply, the liquid sterilizer, is supplied to the equipment from a relative source which can be external, or, as in the present case, internal and forming a part of the instrument 3 to be cleansed, in which case, for instance where the instrument is a turbine drill or a drive handpiece, the source is one and the same as the passage 5i through which spray water reaches the instrument during normal operation.

Similarly, means by which to generate turbulence form a part of the instrument 3, and can be one and the same as the air passage 4i of the instrument itself.

Again, the instrument 3 might afford one internal passage only, i.e. passages 4i and 5i coinciding, in which case the liquid sterilizing medium 2 and means of generating turbulence may be supplied alternately in pulsed mode.

Clearly, given that in normal practice a number of instruments 3 will be sterilized and cleansed at once, those instruments 3 affording an internal water passage 5i through which to supply liquid sterilizer can also serve as an external source of the liquid for instruments not affording any such passage; the same logic applies in the case of the means of generating turbulence.

In order to enhance the cleansing action produced by turbulent flow, it is envisaged that the liquid sterilizer 2 will also contain detergent means.

To advantage, the liquid steriliser 2 utilized will be glutaraldehyde supplemented with surface active agents; in addition to boosting the germicidal properties of the glutaraldehyde, the pH-value of which is acid-weak, such agents produce a cleansing action on the surfaces of the instruments 3 with which they are brought into contact.

An initial step of disinfection or sterilization, say, lasting 3...5 minutes or at least 5 hours, respectively, is followed by a rinsing step that serves to flush away the soiled liquid sterilizer.

The rinse is produced with a stream of clean water supplied in exactly the same manner as indicated for the liquid sterilizer 2, and continuing for a given duration, say of 5...10 minutes following a simple disinfection, or 15...30 minutes following a full sterilization.

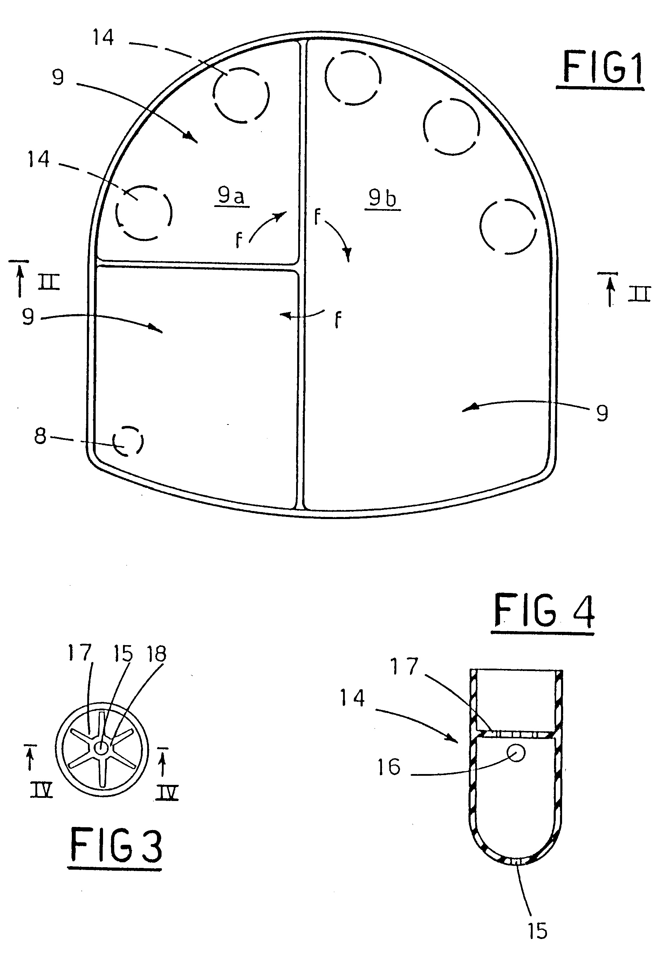

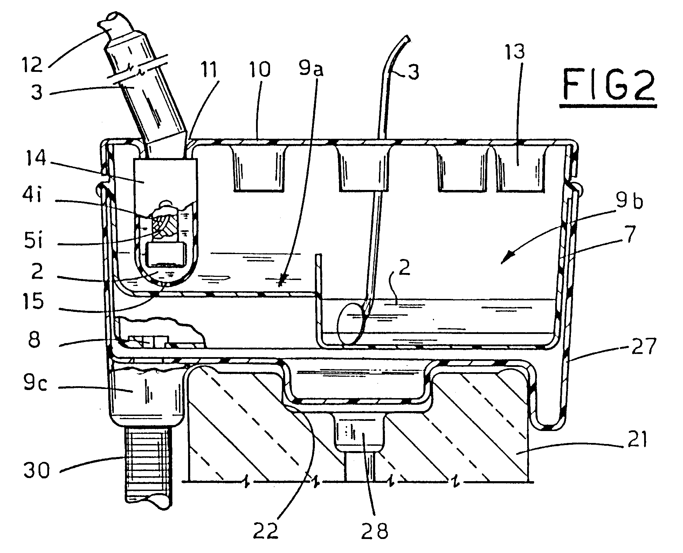

Figs 1 and 2 show the preferred embodiment of the equipment, which comprises a first container 7 provided with a bottom outlet 8 and fitted to the drinking glass holder 22 of the existing surgery apparatus 21 by way of a second container 27 the shape of which is matched to the first container; the holder 22 is provided with a conventional waste outlet 28. The bottom of the first container 7 is embodied in such a manner as to create a plurality of bowls 9 through which the liquid sterilizer 2 passes by overflowing from one to the next, before discharging ultimately from the outlet 8 (fig 2, and arrows f in fig 1).

To advantage, the bowls 9 are of dissmilar depth and disposed at different heights, for a reason that will become apparent in due course.

In the example illustrated, the container 7 is covered over by a lid 10 affording a plurality of holes or sockets 11 designed to accommodate the instruments 3, which are therefore supported in a substantially upright position; accordingly, only those parts of dental surgery instruments that effectively require sterilization need be immersed, while sheaths 12 or other parts liable to damage can be kept free of direct contact with the liquid sterilizer 2.

With specific reference to figs 1 and 2, the source of liquid sterilizer 2 consists in a spray water passage 5i conventionally incorporated into certain dental surgery instruments 3 such as the turbine drill and drive handpiece (see fig 2).

During the sterilization and cleansing cycle, these passages 5i are used to supply sterilizer 2 to the container, rather than conveying water as occurs during normal operation.

As regards the means of generating turbulence, these are provided by the spray air passage 4i with which such instruments 3 are also provided.

In the example of fig 2, the container 7 will be seen to incorporate two bowls 9a and 9b, whilst the sockets 11 formed in the lid 10 consist in tubular projections that are directed down into the inside of the container 7. The transverse dimensions of the bowls 9 are visibly dissimilar, the smaller bowl 9a being set higher than the larger bowl 9b, in such a way that liquid collecting in the former will spill over into and fill the latter to the level illustrated, the reason for which will become apparent in due course.

14 denotes a cap fitted over each of the tubular projections 13 above the smaller bowl 9a (see also figs 3 and 4) which is provided with a hole 15 at bottom of smaller bore than that of the internal passage 5i, and at least one radial hole 16 set at a higher position, of larger bore than that of the internal passage 5i. Located at a level above the radial hole 16, the cap 14 incorporates a plurality of centrally converging flexible elements 17 which appear in figs 3 and 4 as flat tongues lying in a plane disposed transverse to the longitudinal axis of the cap; in their normal, unflexed position, the tongues create a central opening 18 of transverse dimensions less than those of a typical surgery instrument 3 affording internal fluid passages 4i and 5i.

To reiterate, the liquid sterilizer 2 also contains detergent means, and to advantage, will consist in glutaraldehyde supplemented with surface active agents that serve the dual purpose of enhancing the germicidal efficiency of the glutaraldehyde and functioning as a cleanser.

To effect sterilization and cleansing of dental surgery instruments 3, soiled instruments 3 are first inserted through the sockets 11 of the lid 10 into the container 7, to the point of coming to rest substantially on the bottom of the deeper bowl 9b. The sockets 11 provided with caps 14 serve to accommodate those instruments 3, such as the turbine drill, having internal spray water and air passages; this type of instrument does not rest on the bottom of the relative bowl 9a, but is forced through the tongues 17, which flex and assist in holding the tip, preferably at a given distance from the bottom of the relative cap 14 as shown in fig 2. Glutaraldehyde 2 is now pumped through the passage 5i of each instrument 3 held by a set of tongues 17, and the relative caps 14 begin to fill.

The liquid 2 emerges immediately from the bottom hole 15, though given the aforementioned difference in bore between this hole and the passage 5i of the instrument, the liquid also fills the cap 14 until reaching the upper hole 16 and spilling out into the bowl 9a; the liquid 2 subsequently overflows from this bowl 9a into the second bowl 9b, which normally will be occupied by a given number of instruments 3, at least one of which incorporating an air supply passage, e.g. a chip blower. From the second bowl 9b, the liquid 2 overflows finally to the bottom level and gains the outlet 8; this third and last level will generally take the form of a further bowl 9c, incorporating the outlet 8, from which the soiled sterilizer is evacuated through a hose 30 attached to the suction pump of the surgery apparatus.

At the same time, air is also pumped through all of the instruments 3 provided with passages 4i (in both bowls 9a and 9b), and its emergence causes the glutaraldehyde to bubble; accordingly, a turbulent flow is set up in the liquid sterilizer 2 that has the effect of producing a mechanical cleansing action on the internal and/or external surfaces of the instruments 3 exposed to the turbulence.

As far as regards the instruments 3 occupying the caps 14, turbulence here is particularly strong, and the glutaraldehyde 2 will foam as a result of the detergent action produced by the surface active agents on parts with which they come into contact. Foam cannot rise through the sockets 11, however, as it is broken up and retained by the tongues 17.

In the case of the turbine drill, for instance, which incorporates a second air passage supplying pneumatic power for rotation of the turbine (not illustrated in the drawings), this inlet can also be activated to increase turbulence still further and ensure that the sterilizer 2 is brought into contact with internal parts, which thus become thoroughly cleansed.

The instruments 3 immersed in the glutaraldehyde 2 occupying the larger bowl 9b are also exposed to turbulence, generated by the chip blower, for example, and their immersed surfaces likewise undergo a thorough mechanical cleansing action.

It will be observed that, in the situation thus described, one has a source of sterilizer 2 derived internally of certain instruments 3 (e.g. a turbine drill) that serves simultaneously as an external source for other implements such as scale removers, mirrors etc., and the same is true as regards the means for generating turbulence, in this instance, with the turbine drill supplying air internally for its own cleansing, whilst the chip blower serves both as an internal source for its own cleansing and as an external source for that of other instruments and implements having no air supply (scale removers, mirrors, etc...).

## Claims

1. Apparatus for the sterilization and the mechanical cleansing of medical, surgical and dental surgery instruments such as turbine drills and microdrive handpieces, associable with a dental surgery apparatus provided by waste discharging points such as a spittoon bowl and an oral rinse drinking glass holder both provided with respective outlet discharging ducts, said apparatus comprises a receptacle (6, 7) for accommodating instruments (3) for sterilization and cleansing, that can be positioned over one of said waste discharging points, said receptacle being provided by a covering lid (10) provided with sockets (11) for receipt of the instruments (3),
characterized by the fact that
- a plurality of the sockets (11) comprises tubular projections (13) extending into the receptacle which are ensheated at the bottom by a cap (14), which can encompass an instrument (3), at the bottom of the cap a hole is provided which has a smaller bore than at least one radial hole (16), positioned higher in the side wall at a given distance from the bottom hole (15), so that an instrument provided with water passages for a liquid sterilizing medium and air passages for generating turbulence in the liquid sterilizing medium can produce a mechanical cleansing action on the internal and external surfaces of the instrument when accommodated by the cap;
- said cap (14) further comprises a plurality of flexible elements (17) located above the level of the radial hole (16) and extending toward the centre of the cap in such a way as to form an opening (18), which can retain an instrument, when inserted in the cap, and wherein the size and shape of the flexible elements (17) are such that any foam which may form internally of the receptacle is prevented from escaping through the lid (10).

2. Appartus according to claim 1, **characterized by the fact** that the receptacle (7) is provided at the bottom by a plurality of bowls (9) of different depth and communicating one to the other, said liquid sterilizing medium overflowing from one bowl to the next, at least one bowl (9a) being in receipt of the liquid sterilizing medium.

## Patentansprüche

1. Vorrichtung für die Sterilisation und die mechanische Reinigung medizinischer, chirurgischer und zahnärztlicher Instrumente, wie Turbinenbohrer, feingesteuerte Handstücke, anwendbar mit einem zahnärztlichen Gerät, versehen mit Ablaufpunkten wie zum Beispiel einem Speibecken und einem Spülglashalter, beide versehen mit entsprechenden Abflussleitungen, wobei die genannte Vorrichtung ein Aufnahmegefäss (6, 7) zur Aufnahme von Instrumenten (3) zwecks Sterilisation und Reinigung enthält, das über einem der genannten Ablaufpunkte positioniert werden kann, und wobei das genannte Aufnahmegefäss mit einer Abdeckplatte (10) versehen ist, diese ausgestattet mit Sitzen (11) zur Aufnahme der Instrumente (3), **dadurch gekennzeichnet**, dass
- eine Anzahl von Sitzen (11) rohrförmige Ansätze (13) enthält, die sich in das Innere des Aufnahmegefässes erstrecken und an ihrem unteren Ende von einer Kappe (14) umschlossen sind, welche ein Instrument (3) umfassen kann, wobei am Boden der Kappe eine Bohrung vorgesehen ist, welche eine kleinere Öffnung aufweist als wenigstens eine radiale Bohrung (16), angeordnet weiter oben in der Seitenwand mit einem bestimmten Abstand von der Bodenbohrung (15), so dass ein Instrument, versehen mit Wasserleitungen für ein flüssiges Sterilisiermittel und Luftleitungen zur Erzeugung von Turbulenzen in dem flüssigen Sterilisiermittel eine mechanische Reinigungswirkung an den inneren und äusseren Flächen des Instrumentes erzeugen kann, wenn dieses in der Kappe angeordnet ist;
- die genannte Kappe (14) enthält weiter eine Anzahl von flexiblen Elementen (17), die oberhalb der Höhe der radialen Bohrung (16) angeordnet sind und sich zur Mitte der Kappe hin erstrecken, und zwar auf solche Weise, dass eine Öffnung (18) gebildet wird, die ein Instrument festhalten kann, wenn dieses in die Kappe eingesetzt ist, und wobei die Grösse und die Form der flexiblen Elemente (17) solche sind, dass jeglicher Schaum, der sich im Inneren des Aufnahmegefässes bilden kann, am Austreten aus der Abdeckplatte (10) gehindert wird.

2. Vorrichtung nach Patentanspruch 1, **dadurch gekennzeichnet,** dass das Aufnahmegefäss (7) an seinem Boden mit einer Anzahl von Wannen (9) mit unterschiedlichen Tiefen versehen ist, die miteinander in Verbindung stehen, wobei das genannte flüssige Sterilisiermittel von einer Wanne zu der nächsten überfliesst, und wobei wenigstens eine Wanne (9a) ständig das flüssige Sterilisiermittel aufnimmt.

## Revendications

1. Appareil destiné à la stérilisation et au nettoyage mécanique d'instruments médicaux, chirurgicaux et dentaires, tels que des turboroulettes et des pièces à main de micromoteurs, à associer à un appareil de chirurgie dentaire prévoyant des points de déchargement des rebuts et des eaux usées, tels qu'un crachoir et un support de verre à eau destiné au rinçage oral, tous les deux pourvus de respectifs conduits de décharge de sortie, ledit appareil comprenant un récipient (6, 7) destiné a loger les instruments (3) pour la stérilisation et le nettoyage, lequel peut être positionné en correspondance de l'un desdits points de déchargement des rebuts, ledit récipient étant pourvu d'un couvercle de couverture (10) ayant des logements (11) destinés à recevoir les instruments (3),
caractérisé:
- en ce qu'une pluralité desdits logements (11) comporte des saillies tubulaires (13) s'tendant dans le récipient lesquelles sont entourées au fond par un capuchon (14) contenant un instrument (3), sur le fond dudit capuchon étant prévu un trou qui a une ouverture plus petite qu'au moins un trou radial (16) positionné plus haut dans la paroi latérale à une distance donnée du trou de fond (15), de sorte qu'un instrument pourvu de passages pour l'eau destinés au passage d'un moyen de stérilisation liquide et de passages pour l'air susceptibles d'engendrer de la turbulence dans le moyen de stérilisation liquide est en mesure de produire une action de nettoyage mécanique sur les surfaces intérieures et extérieures de l'instrument quand ce dernier est logé dans le capuchon;
- en ce que ledit capuchon (14) comporte en outre une pluralité d'éléments flexibiles (17) disposés au-dessus du niveau du trou radial (16) et s'étendait vers le centre du capuchon, de manière à former une ouverture (18) capable de retenir un instrument quand celui-ci est inséré dans le capuchon, et où la forme et la dimension des éléments flexibiles (17) sont en mesure d'empêcher l'échappement à travers le couvercle (10) de tout type d'écume se formant éventuellement à l'intérieur du récipient.

2. Appareil selon la revendication 1, caractérisé en ce que le récipient (7) est pourvu au fond d'une pluralité de cuvettes (9) de profondeurs différentes et en communication les unes avec les autres, ledit moyen de stérilisation liquide débordant d'une cuvette dans la suivante, au moins l'une (9a) des cuvettes recevant le moyen de stérilisation liquide.
